(19) Europäisches Patentamt
European Patent Office
Office européen des brevets

(11) EP 2 957 559 A1

# (12) EUROPEAN PATENT APPLICATION

(43) Date of publication:
**23.12.2015 Bulletin 2015/52**

(21) Application number: **14305933.5**

(22) Date of filing: **18.06.2014**

(51) Int Cl.:
***C07D 231/54*** *(2006.01)* ***C07D 271/04*** *(2006.01)*
***C07D 495/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **L'Universite de Strasbourg
67000 Strasbourg (FR)**
- **Centre National de la Recherche Scientifique
75016 Paris (FR)**
- **COMMISSARIAT A L'ENERGIE ATOMIQUE ET AUX
ENERGIES ALTERNATIVES
75015 Paris (FR)**

(72) Inventors:
- **Taran, Frédéric
91190 GIF SUR YVETTE (FR)**
- **Wagner, Alain
67000 STRASBOURG (FR)**
- **Kolodych, Sergii
91400 ORSAY (FR)**
- **Koniev, Oleksandr
67000 STRASBOURG (FR)**

(74) Representative: **Tezier Herman, Béatrice
Cabinet Becker & Associés
25, rue Louis le Grand
75002 Paris (FR)**

## (54) Iminosydnone derivatives for conjugation and release of compounds of interest

(57) The present invention relates to the use of iminosydnone compounds in processes for the preparation of conjugates of two compounds of interest. The invention further relates to the use of said iminosydnone compounds in a process for releasing a third compound of interest. The invention finally relates to said new iminosydnone compounds.

EP 2 957 559 A1

## Description

### Introduction

The present invention relates to the use of iminosydnone compounds in processes for the preparation of conjugates of two compounds of interest. The invention further relates to the use of said iminosydnone compounds in a process for releasing a third compound of interest. The invention finally relates to said new iminosydnone compounds.

### Background of the invention

The present invention relates to the domain of click chemistry (bioorthogonal reaction) and of bioconjugation of compounds of interest. Five main approaches are currently available for metal-free coupling two different compounds of interest (● and ■). These approaches are presented on the scheme below. Among these approaches only Tetrazine/Cyclooctene reaction (approach 2) has a modification that allows simultaneous coupling of two entities and release of the third compound *(*Angew. Chem. Int. Ed. 2013, 52, 14112-14116).

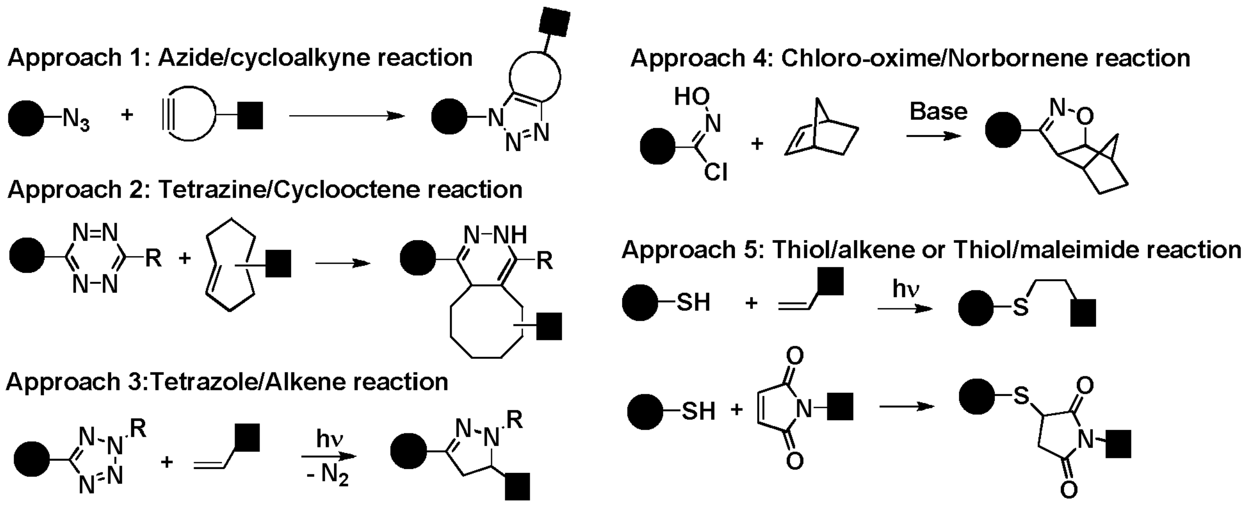


The inventors of the present application recently discovered that reaction of a sydnone and an alkyne could be efficiently used to perform efficient bioorthogonal reactions (Kolodych et al. Angew. Chem. Int. Ed. 2013, 52, 12056-12060).

Bioconjugation reactions and cleavage reactions, for instance bioconjugation and cleavage reactions involving proteins, are often performed in highly dilute conditions, for instance because of the low available amount of the starting biomaterial, or because the solubility of said starting biomaterial is low in the coupling and or cleavage conditions. Furthermore, a high level of bioorthogonality of these reactions is mandatory for applications in the fields of chemical biology and drug release which deal with *in vivo* experiments.

Consequently, it is important in the domain of bioorthogonal reactions to use efficient coupling and cleavable reagents allowing high yields and fast coupling and cleavage kinetics even in the presence of the plethora of chemical functionalities present in biological media. Such reactions are potentially of high interest in several areas; one of them is antibody-drug-conjugate (ADC) cleavage wherein an antibody, such as an anti-tumor antibody, and a drug, such as an anticancer drug, can be selectively released with a specific probe preferably administered in a second step.

The Applicant of the present invention evidenced that the use of iminosydnone derivatives affords a unique process allowing both the ligation and the release of compounds of interest with high efficiency, kinetics and bioorthogonality. These iminosydnone compounds can be advantageously used for the conjugation and the release of compounds of interest.

### Summary of the invention

A first object of the invention is a process for the preparation of a functionalized compound of interest C1 of formula (II):

(II) 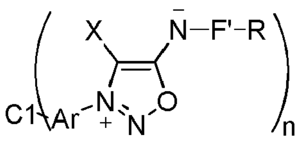 ,

comprising the step of contacting the non-functionalized compound of interest C1 with an iminosydnone of formula (I):

(I) 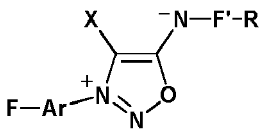 ,

wherein F is a functional group allowing bonding to a compound of interest C1.

Another object of the invention is a functionalized compound of interest C1 of formula (II):

(II) 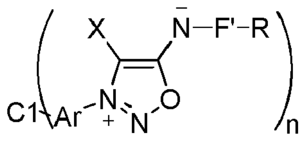 ,

preferably obtained by a process of preparation according to the invention.

Another object of the invention is a conjugate of formula (IV):

(IV) 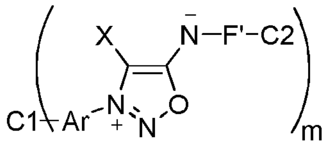 ,

wherein C1 and C2 are compounds of interest.

Another object of the invention is a process for the preparation of a conjugate of formula (IV):

(IV) 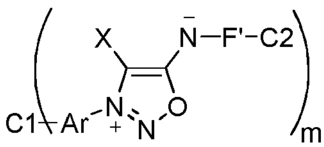 ,

comprising the step of contacting a functionalized compound of interest C1 according to the invention with C2 and/or with a derivative of C2.

Another object of the invention is a process for the preparation of a conjugate of formula (V):

(V) 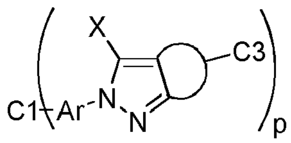

and/or for the release of a compound of interest C2 or a derivative thereof, comprising a step of contacting a compound of interest C3 comprising a strained alkyne moiety of formula (VI):

(VI) 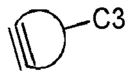

with a functionalized compound of interest C1 of formula (II) or with a conjugate of formula (IV).

Another object of the invention is a conjugate of formula (V):

(V) 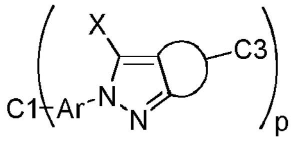,

preferably obtained by a process of preparation according to the invention.

A last object of the invention is an iminosydnone of formula (I):

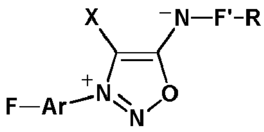 (I),

wherein

X is selected from the group consisting of a hydrogen atom, a halogen atom, an aryl diazo group, an alkyl group, an aryl group, an alkenyl group, an alkynyl group, an alkoxy group, a thioether group and an amino group,
F is a functional group allowing bonding to a compound of interest C1,
F' is a carbonyl group (C=O), a sulfonyl group ($SO_2$) or a phosphoryl group (P=O),
R is selected from the group consisting of an alkyl group, an aryl group, an alkenyl group, an alkynyl group, an alkoxy group, a thioether group, an ether group, an amino group, optionally substituted with one or more groups allowing bonding to a compound of interest C2, and Ar is an aromatic group,
with the proviso that, if F' is a carbonyl group (C=O) and X is a hydrogen atom, then R is not an alkyl group.

## Brief description of the figures

Figure 1: HPLC monitoring of the reaction between iminosydnone Im 5 and the cyclic alkyne TMTH.
Figure 2: HPLC monitoring (b) and linear regression curve (c) for the reaction of Im2 with TMTH (a).
Figure 3: Linear regression curves for the reactions of iminosydnones Im6 and Im7 with TMTH.

## Detailed description of the invention

A first object of the present invention is a process for the preparation of a functionalized compound of interest C1 of formula (II)

(II) 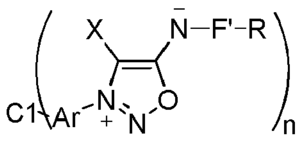,

wherein n is an integer and preferably ranges from 1 to 100, comprising the step of contacting the non-functionalized compound of interest C1 with an iminosydnone of formula (I):

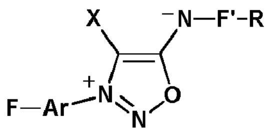
(I) ,

wherein

X is selected from the group consisting of a hydrogen atom, a halogen atom, an aryl diazo group, an alkyl group, an aryl group, an alkenyl group, an alkynyl group, an alkoxy group, a thioether group and an amino group,
F is a functional group allowing bonding to a compound of interest C1,
F' is a carbonyl group (C=O), a sulfonyl group ($SO_2$) or a phosphoryl group (P=O),
R is selected from the group consisting of an alkyl group, an aryl group, an alkenyl group, an alkynyl group, an alkoxy group, a thioether group, and an amino group, optionally substituted with one or more groups allowing bonding to a compound of interest C2, and
Ar is an aromatic group.

A halogen atom is a chlorine, iodine, bromine or fluorine atom. Preferably, a halogen atom is a bromine or a chlorine atom, in particular a chlorine atom.

An alkyl group is a linear, branched or cyclic saturated hydrocarbon group comprising from 1 to 20 carbon atoms. Preferably, the alkyl group according to the invention comprises from 1 to 10, in particular from 1 to 6, carbon atoms. Examples of alkyl groups comprise methyl, ethyl, propyl, isopropyl, butyl, tertbutyl, isobutyl, n-pentyl, n-hexyl and cyclohexyl groups. An alkenyl group (or an alkene) is a linear, branched or cyclic hydrocarbon group comprising from 2 to 20 carbon atoms and comprising at least one C=C double bond. Preferably, the alkenyl group according to the invention comprises from 2 to 10, in particular from 2 to 6, carbon atoms. Examples of alkenyl groups comprise ethylenyl, propylenyl and cyclohexenyl groups.

An alkynyl group (or an alkyne) is a linear, branched or cyclic hydrocarbon group comprising from 2 to 20 carbon atoms and comprising at least one C≡C triple bond. Preferably, the alkynyl group according to the invention comprises from 2 to 10, in particular from 2 to 9, carbon atoms. Examples of alkynyl groups comprise ethynyl, propynyl, octynyl, cyclooctynyl and cyclononynyl groups.

In specific embodiments of the present invention, the alkyl, alkenyl and/or alkynyl groups may be interrupted by at least one heteroatom, preferably independently selected from nitrogen, oxygen and sulphur atoms.

According to the invention, the alkyl groups, alkenes and alkynes are preferably not substituted by an aryl group.

An alkoxy group is an alkyl group, bonded to the rest of the molecule through an oxygen atom.

A thioether group is an alkyl group, bonded to the rest of the molecule through a sulfur atom. An aryl diazo group is a $N_2$ group bonded to an aromatic group. A carboxyl group is a COOH group. A nitro group is a $NO_2$ group.

An amino group is a $NR_1R_2$ group, wherein $R_1$ and $R_2$ are independently selected from the group consisting of hydrogen atoms, aromatic groups and alkyl groups. In an embodiment, at least one of $R_1$ and $R_2$ is an alkyl group. Such an amino group is an alkylamino group. In an embodiment, $R_1$ and $R_2$ are both alkyl groups. Such an amino group is a dialkylaminogroup.

According to the present invention, an aromatic group (or an aryl) is selected from the group consisting of:

- a phenyl group;
- a heteroaromatic group, such as pyridine, thiophene, imidazole, thiazole, pyrazole, pyrole or furane; and
- a polyaromatic group, such as anthracene or phenanthrene;

wherein the phenyl, heteroaromatic and/or polyaromatic group may be optionally substituted with at least one substituent (in addition to the F group when the aromatic is substituted with a F group), preferably selected from the group consisting of a halogen atom, an alkyl group, an alkoxy group, a carboxyl COOH group, a $COOR_3$ group, wherein $R_3$ is an alkyl group, and a nitro $NO_2$ group.

In an embodiment, the aromatic group is an optionally substituted phenyl group, preferably a non substituted phenyl group.

The functional group F is a functional group allowing bonding to a compound of interest C1. For instance, the functional group F may be selected from the group consisting of:

- a carboxylic acid COOH group,
- a thiol SH group,
- a maleimide

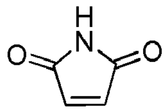

group,

- an activated ester, such as a N-hydroxysuccinimide, a N-hydrophthalimide ester, a perfluorinated ester or an acylurea,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR'')_2$ group, wherein R" is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR"' group, wherein R"' is an alkyl group as defined above, and
- an alkyl group substituted by at least one of the groups above.

The counterion can be any ion appropriate for compensating the charge of the diazonium group, and may be easily chosen by anyone of ordinary skill in the art. For instance, the couterion may be selected from the group consisting of halogenates, $BF_4$-, $NO_3$-, $HSO_4^-$, $PF_6^-$ $CH_3COO^-$, $N(SO_2CF_3)_2^-$, $CF_3SO_3^-$, $CH_3SO_3^-$, $CF_3COO^-$, $(CH_3O)(H)PO_1^-$ and $N(CN)_2^-$.

In an embodiment, X is selected from the group consisting of a hydrogen atom and a halogen atom.

When Ar is an optionally substituted phenyl group, the functional group F is in *meta* or *para* position of the phenyl group, preferably in *para* position.

When Ar is an optionally substituted heteroaromatic or polyaromatic group, the functional group F can be in any position of the aromatic group.

In a preferred embodiment, the functional group F is selected from the group consisting of:

- a carboxylic acid COOH group,
- a thiol SH group,
- a maleimide

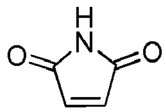

group,

- an activated ester, such as a N-hydroxysuccinimide, a N-hydrophthalimide ester, a perfluorinated ester or an acylurea,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- an isocyanate (-N=C=O) or isothiocyanate (-N=C=S) group,
- a chlorosulfonyl ($-SO_2Cl$) group,
- a -C≡C-C≡N group, and
- an alkyl group substituted by at least one of the groups above.

In a highly preferred embodiment, the functional group F is selected from the group consisting of a carboxylic

acid COOH group, an activated ester, such as a N-hydroxysuccinimide, a N-hydrophthalimide ester, a perfluorinated ester or an acylurea, and an alkyl group substituted by at least one of these groups.

In a preferred embodiment, F' is a C=O group and R is an amino group. Preferably, the R amino group is bonded through the nitrogen atom to the C=O F' group, thus forming a urea moiety.

In an embodiment, if F' is a C=O group and X is a hydrogen atom, then R is not an alkyl group.

Preferably, R is an amino group.

In another preferred embodiment, F' is a C=O group and R is an alkoxy group. Preferably, the R alkoxy group is bonded through the oxygen atom to the C=O F' group, thus forming a carbamate moiety.

In a preferred embodiment, F' is a C=O group, R is an amino or alkoxy group forming a urea or carbamate moiety respectively as disclosed above, and X is a halogen atom, preferably selected from bromine and chlorine atoms.

A group allowing bonding to a compound of interest C2 is selected from the group consisting of: - a carboxylic acid COOH group,

- a thiol SH group,
- a maleimide

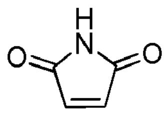

group,
- an activated ester, such as a N-hydroxysuccinimide, a N-hydrophthalimide ester, a perfluorinated ester or an acylurea,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR'')_2$ group, wherein R" is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR"' group, wherein R"' is an alkyl group.

In a preferred embodiment, the group allowing bonding to a compound of interest C2 is selected from the group consisting of: - a carboxylic acid COOH group,
- a thiol SH group,
- a maleimide

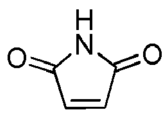

group,
- an activated ester, such as a N-hydroxysuccinimide, a N-hydrophthalimide ester, a perfluorinated ester or an acylurea,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- an isocyanate (-N=C=O) or isothiocyanate (-N=C=S) group,
- a chlorosulfonyl ($-SO_2Cl$) group, and

- a -C≡C-C≡N group.

A compound of interest may be for instance a molecule, such as a fluorophore, for instance rhodamine, a group of atoms comprising at least one radioactive atom ($^{14}C$, $^{3}H$, or $^{131}I$ for instance), a group of atoms of known mass (a mass tag), a ligand, a drug, a therapeutic agent, a biomolecule, such as an antibody, a protein, such as BSA (bovine serum albumin), a DNA fragment, a nanoobject, such as a nanoparticle (*ie* an object or a particle of 0.1 to 1000 nm), or a support, such as a polymer.

In an embodiment, n is from 1 to 50, preferably from 1 to 30. In a specific embodiment, n is 1.

The contacting step of the processes according to the invention may be performed by any technique known in the art, for instance by dissolution and/or dispersion of both compounds in a solvent. The contacting may be performed under stirring, for instance mechanical or magnetic stirring. The contacting may be performed at room temperature, *ie* at a temperature comprised between 18 and 25°C, or at low temperature, *ie* at a temperature inferior to 18°C, or even at high temperature, *ie* at a temperature higher than 25°C, for instance comprised between 25 and 150°C.

The process of preparation of a functionalized compound according to the invention may comprise a preliminary step of covalent bonding of a reactive group to C1, wherein the reactive group is able to react with the functional group F of the iminosydnone compound according to the invention.

In an embodiment, the reactive group is selected from the group consisting of :

- a carboxylic acid COOH group,
- a thiol SH group,
- a maleimide

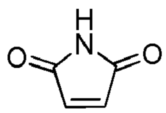

  group,
- an activated ester, such as a N-hydroxysuccinimide, a N-hydrophthalimide ester, a perfluorinated ester or an acylurea,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR'')_2$ group, wherein R" is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR"' group, wherein R'" is an alkyl group.

In a preferred embodiment, the reactive group is selected from the group consisting of:

- a carboxylic acid COOH group,
- a thiol SH group,
- a maleimide

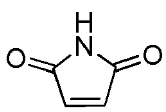

  group,
- an activated ester, such as a N-hydroxysuccinimide, a N-hydrophthalimide ester, a perfluorinated ester or an acylurea,

- an amino (-NRR') group, wherein R and R' are independently hydrogen atoms, alkyl, alkene, alkyne or aryl groups as defined above,
- a hydroxylamine ($-ONH_2$) group,
- a hydrazine ($-NH-NH_2$) group,
- an azido ($-N_3$) group,
- an isocyanate (-N=C=O) or isothiocyanate (-N=C=S) group,
- a chlorosulfonyl ($-SO_2Cl$) group, and
- a -C≡C-C≡N group.

In a highly preferred embodiment, the reactive group is an amino group.

The racemic forms, tautomers, enantiomers, diastereoisomers, epimers, solvates and salts of the compounds according to the invention or used in the processes according to the invention are also part of the scope of the invention.

As examples of salts may be cited acid addition salts, such as hydrochloric, hydrobromic, hydroiodic, phosphoric, and the like. Representative examples of suitable organic acids include formic, acetic, trichloroacetic, trifluoroacetic, propionic, benzoic, cinnamic, citric, fumaric, maleic, methanesulfonic and the like. Further examples of pharmaceutically acceptable inorganic or organic acid addition salts include the pharmaceutically acceptable salts listed in J. Pharm. Sci. 1977, 66, 2, and in Handbook of Pharmaceutical Salts: Properties, Selection, and Use edited by P. Heinrich Stahl and Camille G. Wermuth 2002.

Another object of the invention is a functionalized compound of interest C1 of formula (II):

(II) 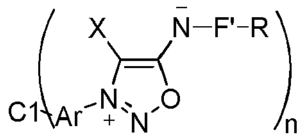

which may be obtained by the process according to the invention.

Another object of the invention is a conjugate of formula (IV):

(IV) 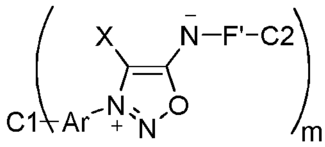,

wherein C1 and C2 are compounds of interest as described above and m is an integer and preferably ranges from 1 to 100. In an embodiment, m is from 1 to 50, preferably from 1 to 30. In a specific embodiment, m is 1. The conjugate of formula (IV) according to the invention may be prepared for instance by contacting a functionalized compound of interest of formula (II) as described above with C2 or with a derivative of C2. A "derivative of C2" according to the present embodiment is any chemical compound comprising a C2 moiety or a moiety that is able to reform the C2 moiety when reacting according to the present process. One of ordinary skill in the art is able to choose (and if necessary prepare) the appropriate derivative of C2 for obtaining the desired conjugate of formula (IV) depending on the chemical structure of C2, R and F'.

Another object of the invention is a process for the preparation of a conjugate of formula (IV) as described above, comprising the step of contacting a functionalized compound of interest of formula (II) with C2 and/or with a derivative of C2.

Another object of the invention is a process for the preparation of a conjugate of formula (V):

(V) 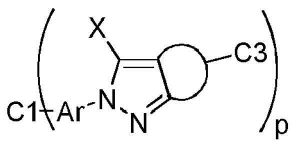,

wherein p is an integer preferably ranging from 1 to 100, C1 is a first compound of interest, and

(VI) 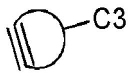

is a third compound of interest C3 comprising a strained alkyne moiety and/or for the release of a compound of interest C2 or a derivative thereof, comprising a step of contacting a compound of interest C3 comprising a strained alkyne moiety, preferably a strained cyclic alkyne moiety, in particular a cyclooctyne moiety, of formula (VI), with a functionalized compound of interest of formula (II) or with a conjugate of formula (IV) according to the invention.

In an embodiment, p is from 1 to 50, preferably from 1 to 30. In a specific embodiment, p is 1.

In a preferred embodiment, the process according to the invention comprises the step of contacting a compound of interest C3 comprising a strained alkyne moiety, preferably a strained cyclic alkyne moiety, in particular a cyclooctyne moiety, of formula (VI)

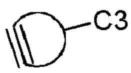

with a conjugate of formula (IV), and simultaneously allows the preparation of a conjugate of formula (V) and the release of a derivative of C2 of formula (VII):

(VII) 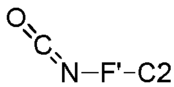.

The process for the preparation of a conjugate of formula (V) may comprise a preliminary step of covalent bonding of a strained alkyne moiety to the compound of interest C3.

Another object of the invention is a conjugate of formula (V):

(V) 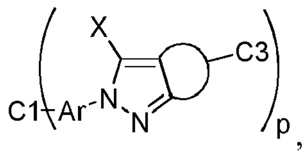,

said conjugate being preferably obtained by the process according to the invention.

Another object of the invention is a process for releasing a compound of interest C2 or a derivative thereof, for instance a derivative of formula (VII):

(VII) 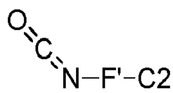,

comprising a step of contacting a conjugate of formula (IV):

(IV) 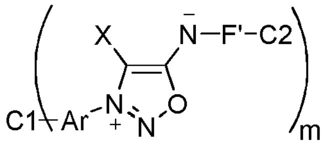

as described above with a compound comprising a strained alkyne moiety, preferably a cyclic alkyne moiety, in particular a cyclooctyne moiety, for instance BCN (bicyclononyne).

Another object of the invention is an iminosydnone of formula (I):

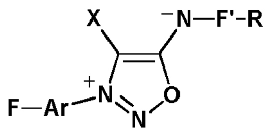 (I),

wherein

X is selected from the group consisting of a hydrogen atom, a halogen atom, an aryl diazo group, an alkyl group, an aryl group, an alkenyl group, an alkynyl group, an alkoxy group, a thioether group and an amino group,
F is a functional group allowing bonding to a compound of interest C1,
F' is a carbonyl group (C=O), a sulfonyl group ($SO_2$) or a phosphoryl group (P=O),
R is selected from the group consisting of an alkyl group, an aryl group, an alkenyl group, an alkynyl group, an alkoxy group, a thioether group, an ether group, an amino group, optionally substituted with one or more groups allowing bonding to a compound of interest C2, and
Ar is an aromatic group,
wherein if F' is a carbonyl group and X is a hydrogen atom, then R is not an alkyl group.

In an embodiment, the iminosydnones according to the invention are selected from the group consisting of:

- (2-(4-hydroxyphenyl)-1,2,3-oxadiazol-2-ium-5-yl)(p-tolylcarbamoyl)amide ;
- (2-(4-carboxyphenyl)-1,2,3-oxadiazol-2-ium-5-yl)(p-tolylcarbamoyl)amide ; and
- ((4-(ethoxycarbonyl)phenyl)carbamoyl)(2-(4-hydroxyphenyl)-1,2,3-oxadiazol-2-ium-5-yl)amide.

The iminosydnone compounds of the invention can be used advantageously in order to couple both compounds of interest C1 and C3 and/or release the compound of interest C2.

In an embodiment, C1 is a nanoobject or a biomolecule such as an antibody, C2 is a therapeutic agent and C3 is a fluorophore or a group of atoms comprising at least one radioactive atom comprising, or functionalized with, a strained alkyne moiety. In this embodiment, the iminosydnone of the invention affords the release of the therapeutic agent and the labeling of the nanoobject or the biomolecule. In a particular embodiment, C1 is an antibody and this embodiment leads to the release of a therapeutic agent from a therapeutic antibody and at the same time the labeling of the antibody, therefore allowing theranostic applications.

The iminosydnones of the invention react very efficiently with strained alkynes, such as cyclic alkynes, in particular cyclooctynes. The coupling reaction implies the formation of a cycloadduct formed by a [3+2] cyclization turning to a stable pyrazole said cycloadduct by retro-Diels Alder reaction, triggering the release of a R-F'-NCO molecule, which in aqueous medium, for instance in water, may spontaneously evolve to R-F'-$NH_2$ through hydrolysis and/or decarboxylation reactions. The reaction with a compound according to the invention with a cycloalkyne is represented as example on the scheme below.

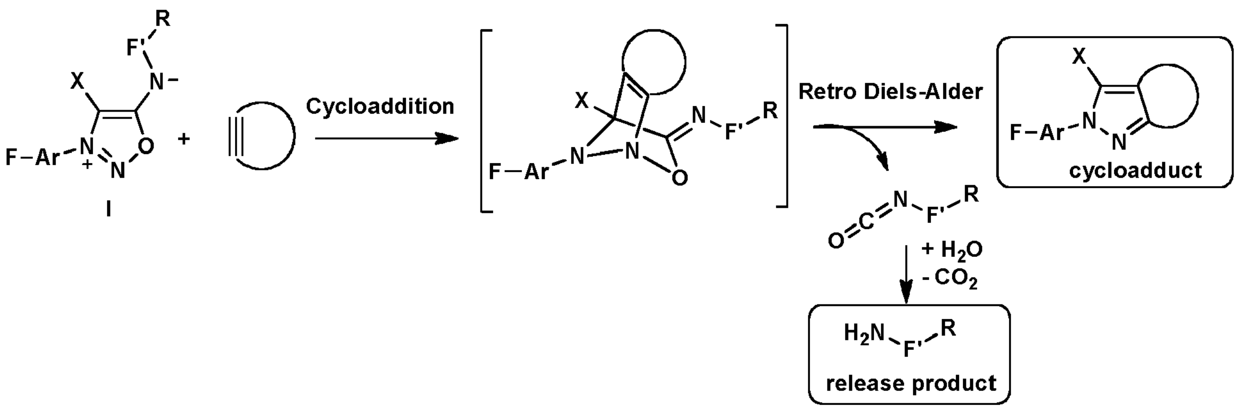


The present compounds allow the efficiency of the reaction to be maintained in biological media, such as culture media, cell lysates or plasma.

The iminosydnones of the invention may be synthesized by any appropriate method known by one of ordinary skill in the art.

For instance, the scheme below presents different synthesis routes for iminosydnones according to the invention.

The Ar groups substituting the nitrogen atom of the iminosydnones may be substituted with a F group. In this scheme, the term NBS designates N-bromosuccinimide.

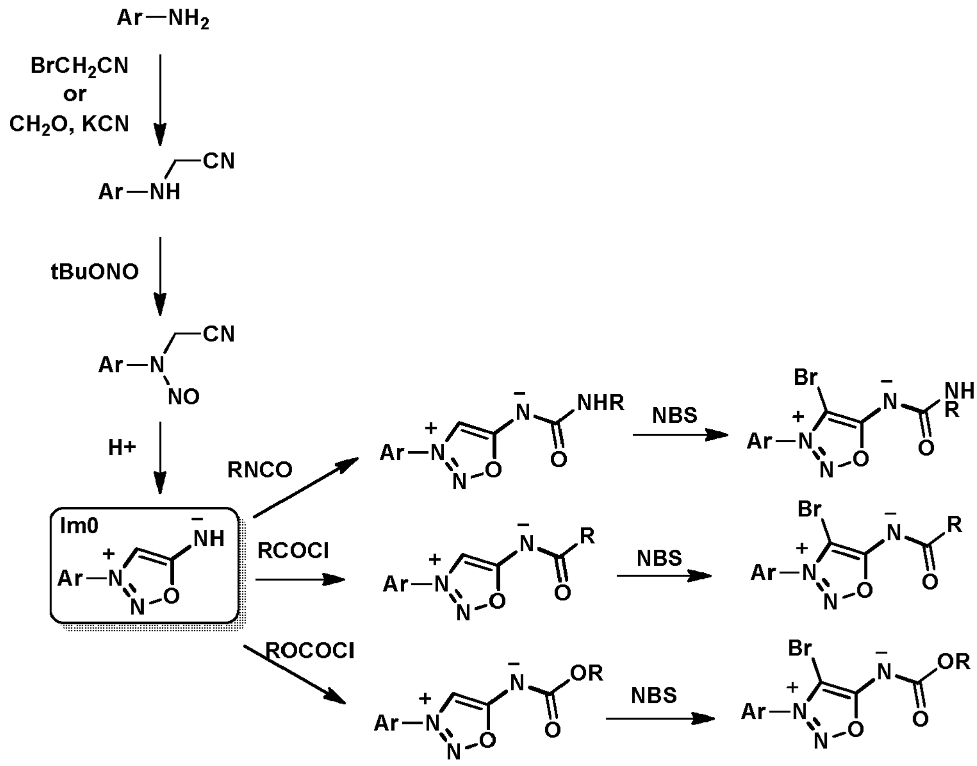


The following examples are provided as illustrative, and not limitative, of the present invention.

**Examples**

**Example** 1: **Synthesis of sydnones according to the invention**

In the following examples, the provided yields are molar yields unless specified differently.

**Im0-1 : 5-amino-3-(4-hydroxyphenyl)-1,2,3-oxadiazol-3-ium chloride**

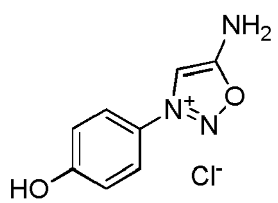

To a solution of *2-((4-hydroxyphenyl)amino)acetonitrile* (2.56 g, 12 mmol) in tetrahydrofuran THF (60 mL) was added amyl nitrite (1.85 eq, 22.2 mmol). The mixture was stirred for 16h at room temperature (r.t.) and then HCl (5 mL, 4M solution in dioxane) was added. The resulting mixture was stirred for 24 h at r.t. The precipitate was collected by filtration and washed with $Et_2O$ and dried to yield pure product as a yellow solid (850 mg, 4.8 mmol, 40%).
**$^1$H NMR (400 MHz, DMSO-$d_6$, $\delta$ ppm):** 9.71 (s, 2 H), 8.48 (s, 1 H), 7.88 (d, J= 9.1 Hz, 2 H), 7.09 (d, J= 9.1 Hz, 2 H).
**$^{13}$C NMR (100 MHz, DMSO-$d_6$, $\delta$ ppm):** 169.1, 162.1, 124.1, 124.0, 116.6, 101.0.

**Im0-2 : 5-amino-3-(4-carboxyphenyl)-1,2,3-oxadiazol-3-ium chloride**

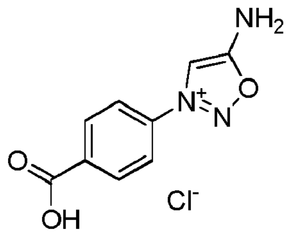

To a solution of *4-((cyanomethyl)amino)benzoic acid* (1.13 g, 6.42 mmol) in THF (60 mL) was added amyl nitrite (1.85 eq, 11.9 mmol). The mixture was stirred for 16h at r.t. and then HCl (5 mL, 4M solution in dioxane) was added. The resulting mixture was stirred for 24 h at r.t. The precipitate was collected by filtration and washed with $Et_2O$ and dried to yield pure product as a white solid (161 mg, 0.67 mmol, 10%).
**$^1$H NMR (400 MHz, DMSO-$d_6$, $\delta$ ppm):** 9.97 (br. s., 2 H), 8.73 (s, 1 H), 8.27 (d, J= 8.6 Hz, 2 H), 8.18 (d, *J* = 8.6 Hz, 2 H).
**$^{13}$C NMR (100 MHz, DMSO-$d_6$, $\delta$ ppm):** 169.4, 165.7, 135.5, 135.1, 131.0, 123.1, 102.7.

**Im** 6 : **(2-(4-hydroxyphenyl)-1,2,3-oxadiazol-2-ium-5-yl)(p-tolylcarbamoyl)amide**

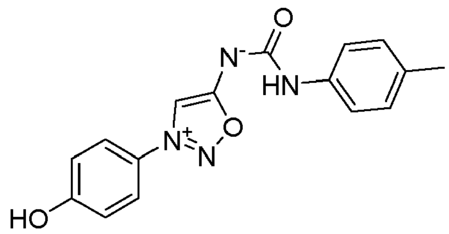

To a solution *of p-tolyl isocyanate* (47 mg, 0.351 mmol) and iminosydnone **Im0-1** S (75 mg, 0.351 mmol) in THF (5 mL) was added a solution of $NaHCO_3$ (1 eq., 30 mg, 0.35 mmol) in $H_2O$ (1 mL). The resulting solution was stirred at room temperature for 14 hours. The organic layer was separated, dried over $MgSO_4$ and evaporated. The residue was purified by semi-preparative HPLC (MeCN/$H_2O$ gradient) to afford **Im6** as a white solid (18.3 mg, 0.059 mmol, 17%).
**$^1$H NMR (400 MHz, DMSO-$d_6$, $\delta$ ppm):** 9.29 (br. s., 1 H), 8.38 (s, 1 H), 7.89 (d, *J* = 9.0 Hz, 2 H), 7.54 (d, J = 8.2 Hz, 2 H), 7.07 - 6.93 (m, J = 6.5, 8.5 Hz, 4 H), 2.22 (s, 3 H).
**$^{13}$C NMR (100 MHz, DMSO-$d_6$, $\delta$ ppm):** 172.2, 161.1, 158.8, 138.5, 129.9, 128.8, 125.3, 123.7, 117.9, 116.4, 102.1, 20.4.

**Im 8: ((4-(ethoxycarbonyl)phenyl)carbamoyl)(2-(4-hydroxyphenyl)-1,2,3-oxadiazo1-2-ium-5-yl)amide.**

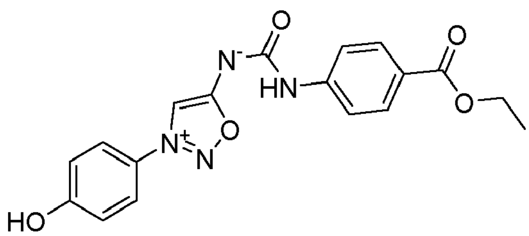

To a solution of *ethyl 4-isocyanatobenzoate* (67 mg, 0.351 mmol) and iminosydnone **Im0-1** (75 mg, 0.351 mmol) in THF (5 mL) was added a solution of $NaHCO_3$ (1 eq., 30 mg, 0.35 mmol) in $H_2O$ (1 mL). The resulting solution was stirred at room temperature for 14 hours. The organic layer was separated, dried over $MgSO_4$ and evaporated. The residue was purified by semi-preparative HPLC (MeCN/$H_2O$ gradient) to afford **Im8** as a yellow solid (16.1 mg, 0.044

mmol, 13%).

**$^1$H NMR (400 MHz, DMSO-d$_6$, δ ppm):** 10.60 (br. s., 1 H), 9.82 (s, 1 H), 8.49 (s, 1 H), 7.91 (d, J= 9.0 Hz, 2 H), 7.84 (d, J= 8.8 Hz, 2 H), 7.77 (d, J= 8.8 Hz, 2 H), 7.02 (d, J= 9.0 Hz, 2 H), 4.27 (q, J = 7.0 Hz, 2 H), 1.30 (t, J= 7.0 Hz, 3 H).

**$^{13}$C NMR (100 MHz, DMSO-d$_6$, δ ppm):** 172.4, 165.5, 161.1, 158.6, 145.6, 130.0, 125.2, 123.7, 122.1, 117.0, 116.3, 102.6, 60.0, 14.2.

**MS (ESI) m/z:** 369.2 [M+H]$^+$.

**Im** 7: **(2-(4-carboxyphenyl)-1,2,3-oxadiazol-2-ium-5-yl)(p-tolylcarbamoyl)amide**

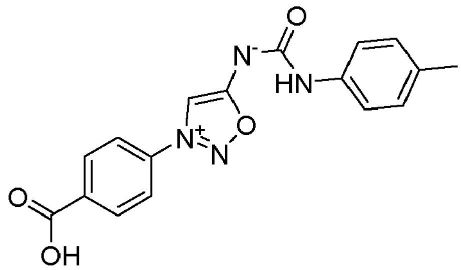

To a solution of *p-tolyl isocyanate* (15 mg, 0.113 mmol) and iminosydnone **Im0-2** (27 mg, 0.113 mmol) in THF (5 mL) was added a solution of $NaHCO_3$ (1 eq., 10 mg, 0.12 mmol) in $H_2O$ (1 mL). The resulting solution was stirred at room temperature for 14 hours. The organic layer was separated, dried over $MgSO_4$ and evaporated. The residue was purified by semi-preparative HPLC (MeCN/$H_2O$ gradient) to afford **Im 7** as a yellow solid (8.6 mg, 0.025 mmol, 22%).

**$^1$H NMR (400 MHz, DMSO-d$_6$, δ ppm):** 9.41 (br. s., 1 H), 8.69 (s, 1 H), 8.21 (s, 4 H), 7.53 (d, J = 8.1 Hz, 2 H), 7.04 (d, J = 8.1 Hz, 2 H), 2.22 (s, 3 H).

**$^{13}$C NMR (100 MHz, DMSO-d$_6$, δ ppm):** 171.8, 165.9, 157.9, 138.1, 136.5, 134.5, 131.0, 130.2, 128.8, 122.6, 118.1, 103.5, 20.3.

**MS (ESI) m/z:** 339.1 [M+H]$^+$.

**Example 2: Kinetic study of the reaction of iminosydnones with cyclic alkynes**

**Experimental:**

Reactions of iminosydnone Im 2 with tetramethylthiacycloheptyne (TMTH) was carried out in PBS buffer (0.1M, pH 7.4) at 100 μM concentration of iminosydnone and 150 μM concentration of cyclooctyne TMTH using the following procedure:

> To 900 μL of PBS buffer were added 10 μL of the solution of benzamide (internal standard, 100 mM in DMSO), 1 μL of the solution of iminosydnone (100 mM in DMSO) and 1.5 μL of the solution of TMTH (100 mM in DMSO). The reaction mixture was injected in HPLC and the conversion was followed by measuring the normalized iminosydnone peak area.

**Results:**

Figures 1 and 2 present the HPLC monitoring of the reaction between the cyclic alkyne TMTH and iminosydnones Im5 and Im 2 respectively. The reaction has been conducted at 100 μM in PBS buffer (pH 7.4). The results indicated an almost complete reaction after 30 min proving that the reaction is fast enough to be useful for bioconjugation and release applications. The presence of a F group on the phenyl group would clearly not affect these results. One can expect similar kinetics for the iminosydnones according to the invention.

**Example 3: Kinetic study of different iminosydnones**

The kinetics of the coupling reaction of different iminosydnones with two cyclic alkynes was studied, according to the following scheme.

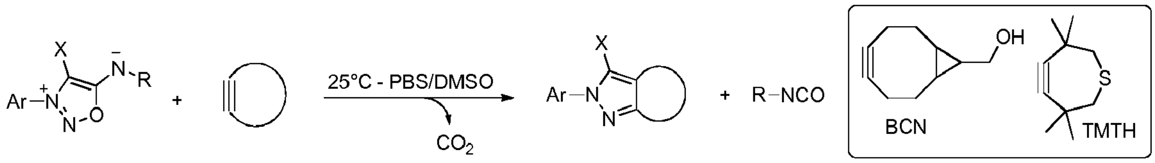


**Experimental:**

Reactions of iminosydnones with BCN (Biclyclononyne) or TMTH were carried out in PBS/DMSO (9:1) mixtures at 100 μM concentration of sydnones and 150 μM concentration of BCN or TMTH using the following procedure:

To 900 μL of phosphate buffered saline (PBS, 100 mM) was added 87.5 μL of DMSO, 10 μL of the solution of benzamide (internal standard, 100 mM in DMSO), 1 μL of the solution of iminosydnone (100 mM in DMSO) and 1.5 μL of the solution of BCN or TMTH (100 mM in DMSO). The reaction mixture was injected in HPLC every 30 min and the conversion was followed by measuring the normalized sydnone peak area.

**Results:**

Table 1 below presents the kinetic constant values K for these reactions, depending on the Ar group, the R group and the X group. Iminosydnones Im 6, 7 and 8 are according to the invention.

**Table 1.** K unit is $M^{-1}.sec^{-1}$, n.d. = not determined. Reactions were conducted with 100 μM of iminosydnones and 150 μM of BCN or TMTH in PBS 0.1 M (pH 7.4) containing 10% DMSO

| **Iminosydnones** | **Ar** | **X** | **R** | **K (BCN)** | **K (TMTH)** |
|---|---|---|---|---|---|
| **Im1** | $C_6H_5$ | H | H | no reaction | n.d. |
| **Im2** | $C_6H_5$ | H | $CH_3CO$- | 0.002 | 2.2 |
| **Im3** | $p CH_3C_6H_4$ | H | $C_6H_5CH_2CO$- | 0.004 | n.d. |
| **Im4** | $p CH_3C_6H_4$ | H | $C_6H_5CH_2$ $CH_2CO$- | 0.034 | n.d. |
| **Im5** | $C_6H_5$ | H | $p CH_3C_6H_4NHCO$- | 0.069 | 43.1 |
| **Im6** | $p OHC_6H_4$ | H | $p CH_3C_6H_4NHCO$- | 0.008 | 4.1 |
| **Im7** | $p CO_2HC_6H_4$ | H | $p CH_3C_6H_4NHCO$- | 0.108 | 61.2 |
| **Im8** | $p OHC_6H_4$ | H | $p CO_2EtC_6H_4NHCO$- | 0.008 | 2.5 |
| **Im9** | $p CH_3C_6H_4$ | H | $C_6H_5CH_2NHCO$- | 0.004 | n.d. |
| **Im10** | $p CH_3C_6H_4$ | Br | $C_6H_5CH_2NHCO$- | 0.060 | n.d. |
| **Im11** | $p CH_3C_6H_4$ | H | $C_6H_5CH_2OCO$- | 0.026 | n.d. |
| **Im12** | $p CH_3C_6H_4$ | Br | $C_6H_5CH_2OCO$- | 0.150 | n.d. |

These results clearly show that the iminosydnones comprising a urea or a carbamate function at F' position and the iminosydnones comprising a halogen atom in position 4 afford the fastest reactions. In particular, 4-bromo-6-carbamate iminosydnones were found to react with the cyclooctyne BCN with a constant of 0.15 $M^{-1}.sec^{-1}$ which allows the reaction to proceed at concentration as low as 1 μM.

Second order reaction rate was determined by plotting ln([A]/[B])/([A] - [B]) versus time and analyzing by linear regression (Equation). Second order rate constant corresponds to the determined slope.

$$\frac{\ln\left(\frac{[A]}{[B]}\right)}{[A]-[B]} = kt + const$$

**Equation.** [A] – concentration of iminosydnones (M); [B] – concentration of BCN (M); t – reaction time (sec); k – reaction rate ($M^{-1} \cdot sec^{-1}$)

Linear regression curves for sydnones Im2, Im6 and Im7 are illustrated in figures 2 and 3.

## Claims

1. Process for the preparation of a functionalized compound of interest C1 of formula (II):

(II) 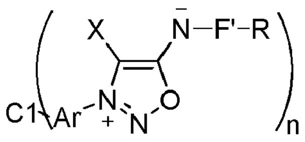 ,

wherein n is an integer from 1 to 100, comprising the step of contacting the non-functionalized compound of interest C1 with an iminosydnone of formula (I):

(I) 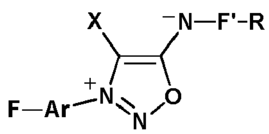 ,

wherein:

X is selected from the group consisting of a hydrogen atom, a halogen atom, an aryl diazo group, an alkyl group, an aryl group, an alkenyl group, an alkynyl group, an alkoxy group, a thioether group and an amino group,
F is a functional group allowing bonding to a compound of interest C1,
F' is a carbonyl group (C=O), a sulfonyl group ($SO_2$) or a phosphoryl group (P=O),
R is selected from the group consisting of an alkyl group, an aryl group, an alkenyl group, an alkynyl group, an alkoxy group, a thioether group, and an amino group, optionally substituted with one or more groups allowing bonding to a compound of interest C2, and
Ar is an aromatic group,
and wherein the alkyl, alkenyl and/or alkynyl groups may be interrupted by at least one heteroatom selected from nitrogen, oxygen and sulphur atoms.

2. Process for the preparation of a functionalized compound of interest C1 according to claim 1, wherein F is selected from the group consisting of:

- a carboxylic acid COOH group,
- a thiol SH group,
- a maleimide

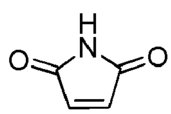

group,
- an activated ester,
- a halogen atom,
- an alkene or alkyne group, optionally interrupted by at least one heteroatom selected among O, N and S,

- an amino (-NRR') group, wherein R and R' are independently hydrogen atoms, alkyl, alkene, alkyne or aryl groups,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR")_2$ group, wherein R" is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR"' group, wherein R"' is an alkyl group, and
- an alkyl group substituted by at least one of said groups.

**3.** Process for the preparation of a functionalized compound of interest C1 according to claim 2, wherein F is selected from the group consisting of a carboxylic acid COOH group, an activated ester and an alkyl group substituted by at least one of these groups

**4.** Process for the preparation of a functionalized compound of interest C1 according to any one of claims 1 or 3, comprising a preliminary step of covalent bonding of a reactive group to C1, wherein the reactive group is able to react with the functional group F of the iminosydnone of formula (I).

**5.** Process for the preparation of a functionalized compound of interest C1 according to claim 4, wherein the reactive group is selected from the group consisting of :

- a carboxylic acid COOH group,
- a thiol SH group,
- a maleimide

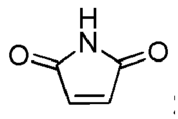

group,
- an activated ester,
- a halogen atom,
- an alkene or alkyne group, optionally interrupted by at least one heteroatom selected among O, N and S,
- an amino (-NRR') group, wherein R and R' are independently hydrogen atoms, alkyl, alkene, alkyne or aryl groups,
- a hydroxylamine ($-ONH_2$) group,
- a hydrazine ($-NH-NH_2$) group,
- an azido ($-N_3$) group,
- a diazonium ($-N_2^+$) group, optionally in presence of a counterion,
- a boronic acid $-B(OR")_2$ group, wherein R" is a hydrogen atom or an alkyl group,
- an isocyanate (-N=C=O) or isothiocyanate (-N=C=S) group,
- a chlorosulfonyl ($-SO_2Cl$) group,
- a -C≡C-C≡N group,
- an aldehyde CHO group,
- a ketone COR"' group, wherein R"' is an alkyl group.

**6.** Process for the preparation of a functionalized compound of interest C1 according to claim 5, wherein the reactive group is an amino group.

**7.** Functionalized compound of interest C1 of formula (II):

(II) 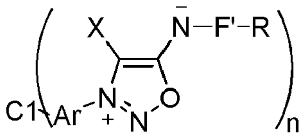,

wherein n is an integer from 1 to 100, C1 is a compound of interest selected from the group consisting of a fluorophore, a group of atoms comprising at least one radioactive atom, a group of atoms of known mass, a ligand, a drug, a therapeutic agent, a biomolecule, a DNA fragment, a nanoobject, or a support,
X is selected from the group consisting of a hydrogen atom, a halogen atom, an aryl diazo group, an alkyl group, an aryl group, an alkenyl group, an alkynyl group, an alkoxy group, a thioether group and an amino group,
F' is a carbonyl group (C=O), a sulfonyl group ($SO_2$) or a phosphoryl group (P=O),
R is selected from the group consisting of an alkyl group, an aryl group, an alkenyl group, an alkynyl group, an alkoxy group, a thioether group, an amino group, optionally substituted with one or more groups allowing bonding to a compound of interest C2, and
Ar is an aromatic group,
wherein the alkyl, alkenyl and/or alkynyl groups may be interrupted by at least one heteroatom selected from nitrogen, oxygen and sulphur atoms, and
wherein the functionalized compound of interest is obtained by the process of claim 2.

**8.** Conjugate of formula (IV):

(IV) 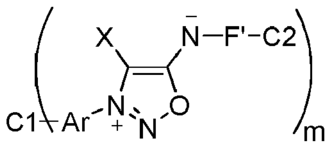,

wherein

m is an integer from 1 to 100,
C1 and C2 are compounds of interest independently selected from the group consisting of a fluorophore, a group of atoms comprising at least one radioactive atom, a group of atoms of known mass, a ligand, a drug, a therapeutic agent, a biomolecule, a DNA fragment, a nanoobject, and a support,
X is selected from the group consisting of a hydrogen atom, a halogen atom, an aryl diazo group, an alkyl group, an aryl group, an alkenyl group, an alkynyl group, an alkoxy group, a thioether group and an amino group,
F' is a carbonyl group (C=O), a sulfonyl group ($SO_2$) or a phosphoryl group (P=O), Ar is an aromatic group, and wherein the alkyl, alkenyl and/or alkynyl groups may be interrupted by at least one heteroatom selected from nitrogen, oxygen and sulphur atoms.

**9.** Process for the preparation of a conjugate according to claim 8, comprising the step of contacting a functionalized compound of interest C1 according to claim 7 with C2 and/or with a derivative of C2, wherein C2 is a compound of interest selected from the group consisting of a fluorophore, a group of atoms comprising at least one radioactive atom, a group of atoms of known mass, a ligand, a drug, a therapeutic agent, a biomolecule, a DNA fragment, a nanoobject, and a support.

**10.** Process for the preparation of a conjugate of formula (V):

(V) 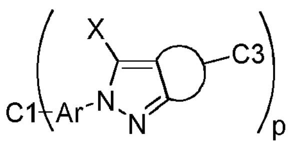,

wherein p is an integer from 1 to 100, C1 is a first compound of interest, and

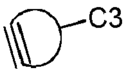 (VI)

is a third compound of interest C3 comprising a strained alkyne moiety and/or for the release of a compound of interest C2 or a derivative thereof, comprising a step of contacting a compound of interest C3 comprising a strained alkyne moiety with a functionalized compound of interest according to claim 7 or with a conjugate according to claim 8, wherein each compound of interest is independently selected from the group consisting of a fluorophore, a group of atoms comprising at least one radioactive atom, a group of atoms of known mass, a ligand, a drug, a therapeutic agent, a biomolecule, a DNA fragment, a nanoobject, and a support.

**11.** Process of preparation according to claim 10, wherein the process comprises a preliminary step of covalent bonding of a strained alkyne moiety to the compound of interest C3.

**12.** Process for releasing a derivative of a compound of interest C2 of formula (VII):

(VII) 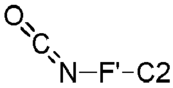,

comprising a step of contacting a conjugate of formula (IV) according to claim 8 with a compound comprising a strained alkyne moiety.

**13.** Iminosydnone of formula (I):

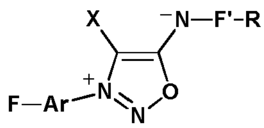 (I),

wherein

X is selected from the group consisting of a hydrogen atom, a halogen atom, an aryl diazo group, an alkyl group, an aryl group, an alkenyl group, an alkynyl group, an alkoxy group, a thioether group and an amino group,
F is a functional group allowing bonding to a compound of interest C1,
F' is a carbonyl group (C=O), a sulfonyl group ($SO_2$) or a phosphoryl group (P=O),
R is selected from the group consisting of an alkyl group, an aryl group, an alkenyl group, an alkynyl group, an alkoxy group, a thioether group and an amino group, optionally substituted with one or more groups allowing bonding to a compound of interest C2, and
Ar is an aromatic group,
wherein the alkyl, alkenyl and/or alkynyl groups may be interrupted by at least one heteroatom selected from nitrogen, oxygen and sulphur atoms,
with the proviso that, if F' is a carbonyl group (C=O) and X is a hydrogen atom, then R is not an alkyl group.

**14.** Iminosydnone according to claim 13, wherein F' is a C=O group and R is an amino group, forming a urea moiety with the F' group, and X is a halogen atom selected from chlorine and bromine.

**15.** Iminosydnone according to claim 13, wherein the iminosydnone is selected from the group consisting of:

- (2-(4-hydroxyphenyl)-1,2,3-oxadiazol-2-ium-5-yl)(p-tolylcarbamoyl)amide),
- (2-(4-carboxyphenyl)-1,2,3-oxadiazol-2-ium-5-yl)(p-tolylcarbamoyl)amide, and
- ((4-(ethoxycarbonyl)phenyl)carbamoyl)(2-(4-hydroxyphenyl)-1,2,3-oxadiazol-2-ium-5-yl)amide.

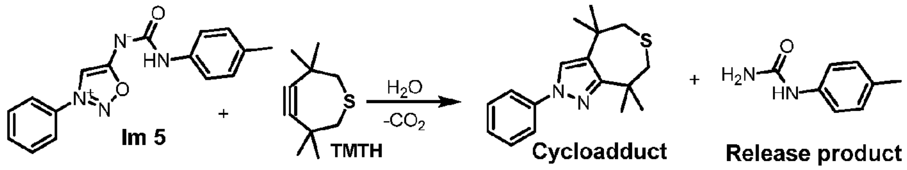


a)

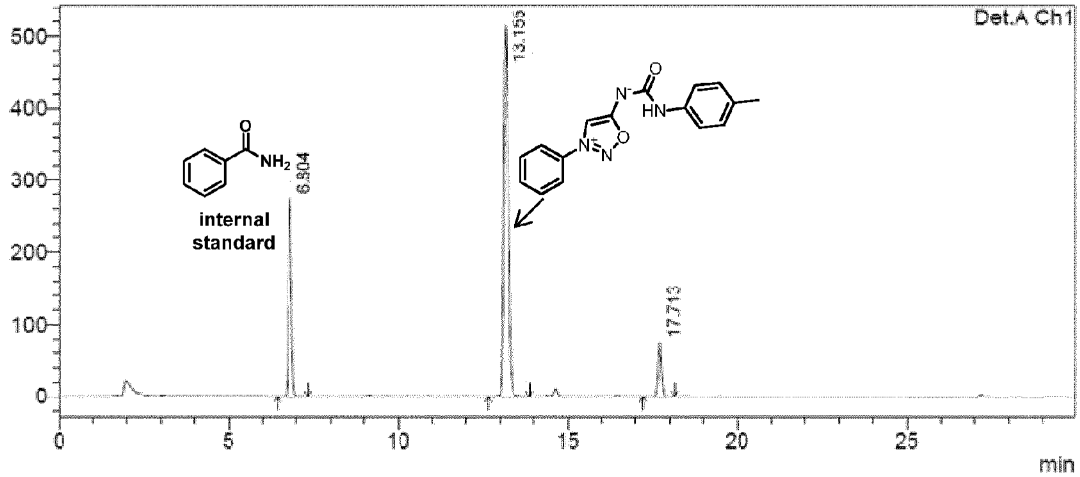


b)

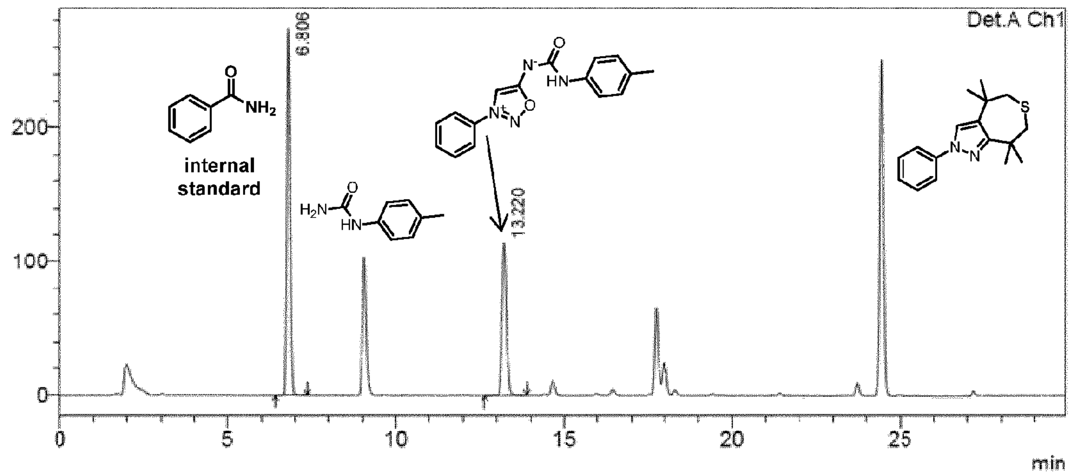


c)

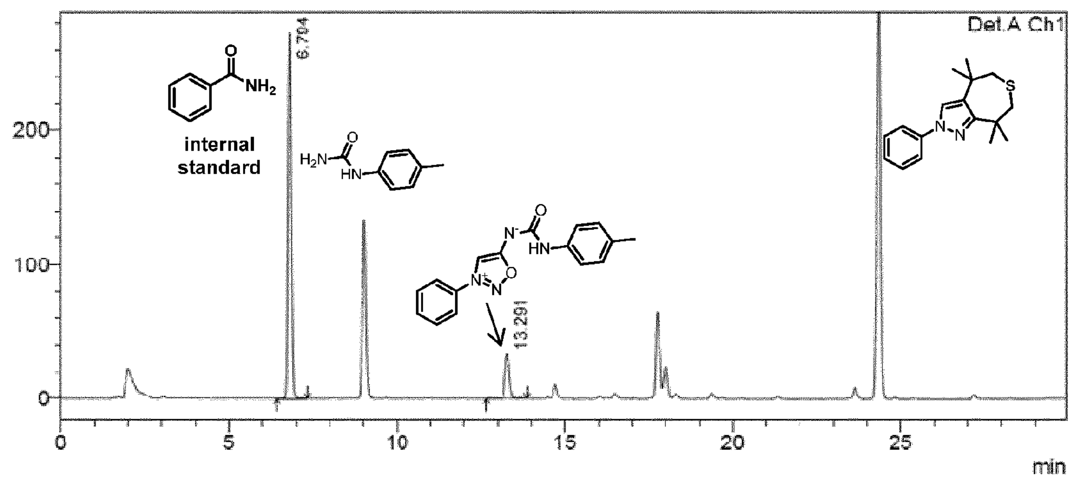


d)

Figure 1

a)

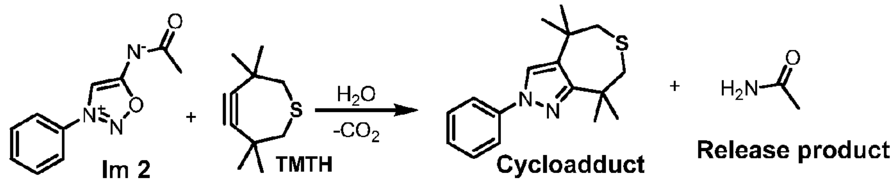

Im 2
TMTH
$H_2O$
$-CO_2$
Cycloadduct
Release product


b)

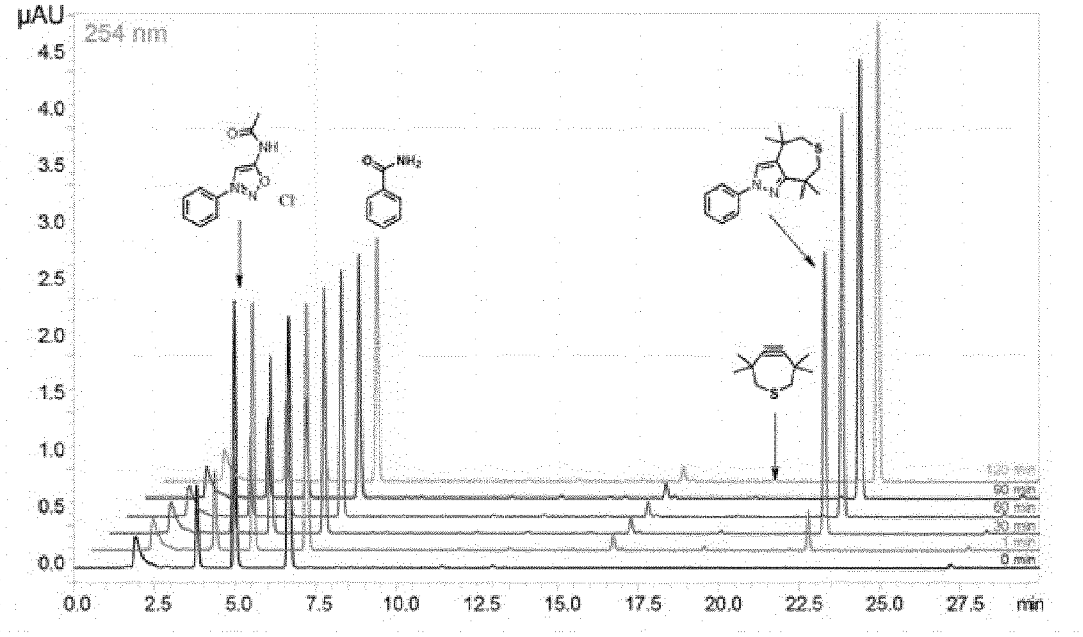

µAU
254 nm
4.5
4.0
3.5
3.0
2.5
2.0
1.5
1.0
0.5
0.0
0.0 2.5 5.0 7.5 10.0 12.5 15.0 17.5 20.0 22.5 25.0 27.5 min
120 min
90 min
60 min
30 min
1 min
0 min


c)

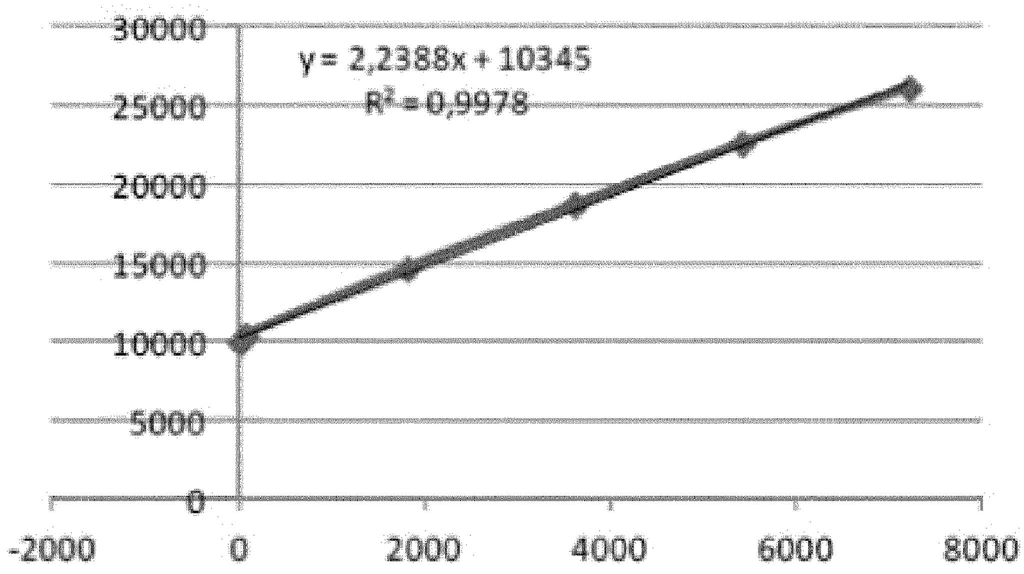

y = 2,2388x + 10345
$R^2$ = 0,9978
30000
25000
20000
15000
10000
5000
0
-2000 0 2000 4000 6000 8000


Figure 2

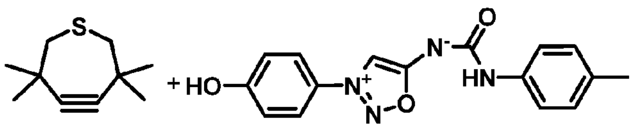

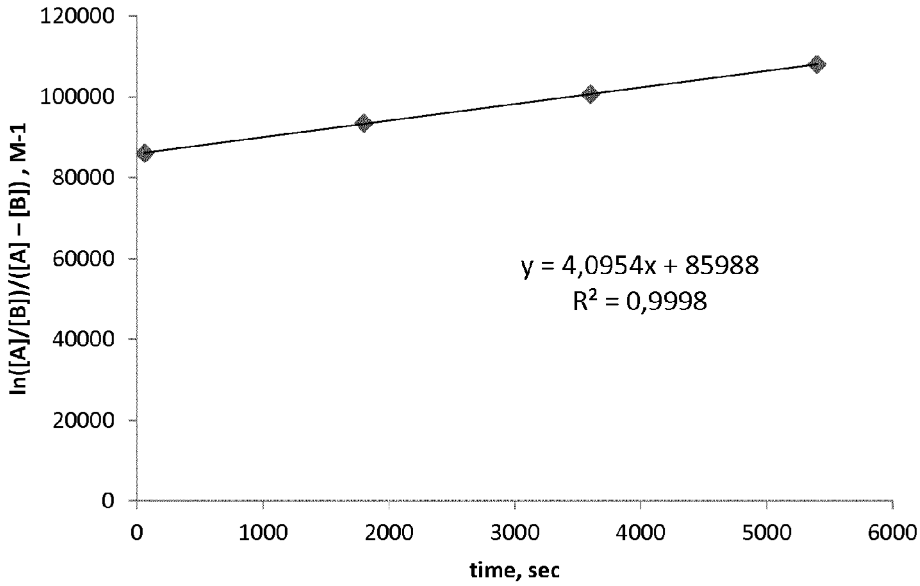
120000
100000
80000
60000
40000
20000
0
0
1000
2000
3000
4000
5000
6000
time, sec
ln([A]/[B])/([A] – [B]) , M-1
y = 4,0954x + 85988
R² = 0,9998

a)

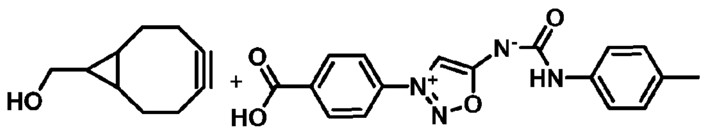

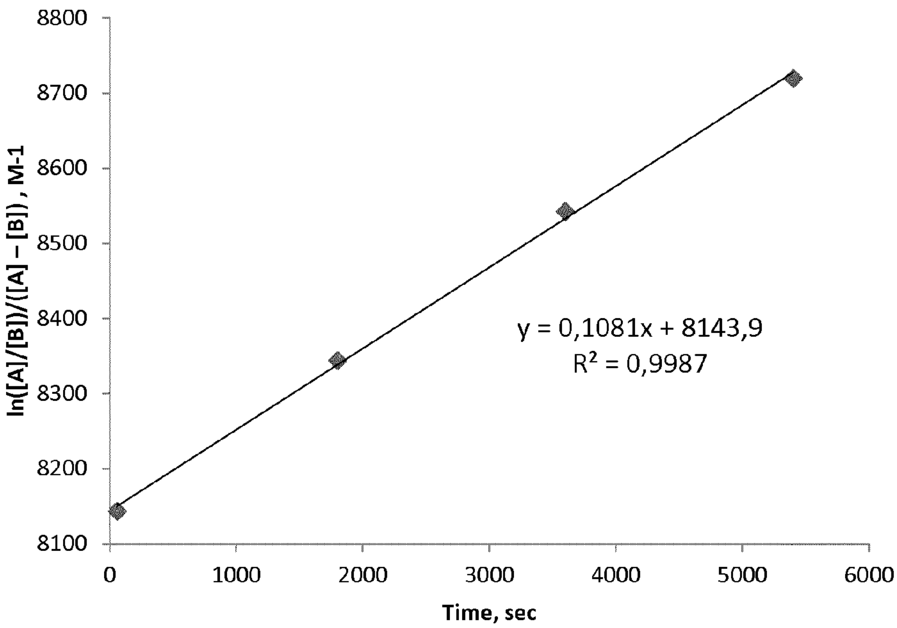
8800
8700
8600
8500
8400
8300
8200
8100
0
1000
2000
3000
4000
5000
6000
Time, sec
ln([A]/[B])/([A] – [B]) , M-1
y = 0,1081x + 8143,9
R² = 0,9987

b)

Figure 3

Europäisches Patentamt
European Patent Office
Office européen des brevets

# EUROPEAN SEARCH REPORT

Application Number
EP 14 30 5933

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | DAENIKER; DRUEY: "Heilmittelchemische Studien In Der Heterocyclischen Reihe. 36. Mitteilung. Über Sydnonimine. II. Herstellung und Eigenschaften von Sydnoniminen mit Acyl-, Carbamoyl- und Thiocarbamoyl-Substituenten am exocyclischen Stickstoffatom", HELV. CHIM. ACTA, vol. 45, no. 7, 1 January 1962 (1962-01-01), pages 2441-2462, XP055152550, ISSN: 0018-019X, DOI: 10.1002/hlca.19620450714 * Page 2442; page 2452, table 6; page 2453, paragraph 2. * | 1,7-9 | INV. C07D231/54 C07D271/04 C07D495/04 |
| X | DATABASE CA [Online] CHEMICAL ABSTRACTS SERVICE, COLUMBUS, OHIO, US; YASHUNSKII, V. G. ET AL: "Sydnones and sydnonimines. XXVI. Reactivity of 3-aryl-sydnonimines", XP002732477, retrieved from STN Database accession no. 1966:59381 * Abstract; CAS-RNs 838-38-0, 838-43-7, 838-44-8, 840-66-4, 842-85-3. * & COLLECTION OF CZECHOSLOVAK CHEMICAL COMMUNICATIONS , 30(12), 4257-71., 1965, | 7,8,13 | TECHNICAL FIELDS SEARCHED (IPC) C07D |

-/--

The present search report has been drawn up for all claims

3

| Place of search | Date of completion of the search | Examiner |
|---|---|---|
| Munich | 14 November 2014 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 30 5933

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online] CHEMICAL ABSTRACTS SERVICE, COLUMBUS, OHIO, US; KAMITANI, TAKASHI ET AL.: "Sydnonimines", XP002732478, retrieved from STN Database accession no. 1971:13164 * Abstract; CAS-RNs 30011-56-4, 30011-57-5. * & JP S45 26086 B (FUJISAWA PHARMACEUTICAL CO., LTD.) 28 August 1970 (1970-08-28) ----- | 7,8,13 | |
| X | DATABASE CA [Online] CHEMICAL ABSTRACTS SERVICE, COLUMBUS, OHIO, US; KAMITANI, TAKASHI ET AL: "Sydnonimines", XP002732479, retrieved from STN Database accession no. 1971:13161 * Abstract; CAS-RN 30039-75-9. * & JP S45 26088 B (FUJISAWA PHARMACEUTICAL CO., LTD.) 28 August 1970 (1970-08-28) ----- | 7,8,13 | TECHNICAL FIELDS SEARCHED (IPC) |
| X | DATABASE CA [Online] CHEMICAL ABSTRACTS SERVICE, COLUMBUS, OHIO, US; KAMITANI, TAKASHI ET AL: "N-Substituted-syndnonimines", XP002732480, retrieved from STN Database accession no. 1971:13160 * Abstract; CAS-RNs 30039-65-7, 30039-66-8, 30039-67-9. * & JP S45 26090 B (FUJISAWA PHARMACEUTICAL CO., LTD.) 28 August 1970 (1970-08-28) ----- -/-- | 7,8,13 | |

The present search report has been drawn up for all claims

3

| Place of search | Date of completion of the search | Examiner |
|---|---|---|
| Munich | 14 November 2014 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 30 5933

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | BROWNE; HARRITY: "Recent developments in the chemistry of sydnones", TETRAHEDRON, vol. 66, no. 3, 16 January 2010 (2010-01-16), pages 553-568, XP027534268, ISSN: 0040-4020 [retrieved on 2009-10-29] * Chapter 4: alkyne cycloadditions. * ----- | 10-12 | |
| X | WALLACE; CHIN: "Strain-promoted sydnone bicyclo-[6.1.0]-nonyne cycloaddition", CHEMICAL SCIENCE, vol. 5, no. 5, 5 February 2014 (2014-02-05), page 1742, XP055142993, ISSN: 2041-6520, DOI: 10.1039/c3sc53332h * The whole document. * ----- | 10-12 | |
| A,D | KOLODYCH ET AL.: "Discovery of Chemoselective and Biocompatible Reactions Using a High-Throughput Immunoassay Screening", ANGEW. CHEM. INT. ED., vol. 52, no. 46, 7 October 2013 (2013-10-07), pages 12056-12060, XP055142984, ISSN: 1433-7851, DOI: 10.1002/anie.201305645 * The whole document. * ----- | 1 | TECHNICAL FIELDS SEARCHED (IPC) |
| | -/-- | | |

The present search report has been drawn up for all claims

3

| Place of search | Date of completion of the search | Examiner |
|---|---|---|
| Munich | 14 November 2014 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 30 5933

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VINATIER ET AL.: "Esterase-activated chromane-sydnonimine prodrug hybrids", NITRIC OXIDE, vol. 15, no. 4, 1 December 2006 (2006-12-01), pages 363-369, XP024919018, ISSN: 1089-8603, DOI: 10.1016/J.NIOX.2006.03.006 [retrieved on 2006-12-01] * Abstract; page 365, figure 2; page 367, figure 7. * ----- | 1 | |
| | | | TECHNICAL FIELDS SEARCHED (IPC) |

The present search report has been drawn up for all claims

3

| Place of search | Date of completion of the search | Examiner |
|---|---|---|
| Munich | 14 November 2014 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Angew. Chem. Int. Ed.,* 2013, vol. 52, 14112-14116
- **KOLODYCH et al.** *Angew. Chem. Int. Ed.,* 2013, vol. 52, 12056-12060
- *J. Pharm. Sci.,* 1977, vol. 66, 2
- Handbook of Pharmaceutical Salts: Properties, Selection, and Use. 2002